# EUROPEAN PATENT APPLICATION

(11) **EP 0 763 390 A2**
(43) Date of publication of application: **19.03.1997**
(21) Application number: 96500122.5
(22) Date of filing: 10.09.1996
(51) Int. Cl.: B09B 3/00, A61L 11/00

(54) **System for treating hospital waste, starting from bags of the said waste**

(30) Priority: 13.09.1995 ES 9501773
(71) Applicant: Rivera Montanes, Carmelo, 01007 Vitoria-Gasteiz (ES)
(72) Inventor: Rivera Montanes, Carmelo, 01007 Vitoria-Gasteiz (ES)
(74) Representative: Urteaga Simarro, José Antonio

(57) **Abstract**

A system which treats hospital waste contained in unitary bags which are housed in cartridges (3). These cartridges are connected to an intake of a unit (26) which includes a sterilization chamber (15), a steam boiler (13) and an area for putting the waste into sacks or bags once they have been treated inside the sterilization chamber. The sacks are ground (triturated) and the material contained is separated into metallic and non-metallic components and treated in an autoclave. The final material collected into bags is composed of pathogen-free waste that can be sent to solid waste plants for further use.

## Description

This invention relates to a system for treating hospital waste, which is usually collected into plastic bags by the cleaning staff of healthcare institutions, such as medical centres, clinics, hospitals, etc.

Until now, and as far as is known, this waste is processed directly from the said bags, without taking into account that this kind of waste usually contains items entailing health risks, which should be neutralized as quickly as possible.

One object of the invention is to provide a system for treating waste of this kind in a fast, ingenious way, neutralizing the waste and collecting it, in an inert form, into bags, for its later treatment as solid urban waste.

Another object of the invention is the installation to carry our the system in question.

In order to achieve these objectives, the invention establishes the incorporation of some steel container cartridges, inside which the bags of waste are stored until all the interior is occupied. These cartridges have some particular characteristics so that they can be re-used later.

Thus, they are equipped with a hermetic lid and an upper area in the shape of a hook, while their lower end is provided with wheels at the ends, with a base separated from the said end which is composed of a trapdoor or gate which can be opened, for example a split wicket gate or butterfly gate.

From the trapdoor or gate to the end of the wheels, there is a tubular portion capable of being housed in the intake of a treatment unit once that its interior has been occupied by the bags of waste.

The treatment unit, which henceforth will be referred to simply as "the unit", is provided with a cylindrical intake into which the above mentioned cartridge is received, with a fast connection system and hermetic sealing. The intake in question is of a similar type to that of the cartridge, with a base separated a certain distance from its upper end, which is also made up of an automatic trapdoor or gate.

The unit includes a steam production chamber, a sterilization chamber and a chamber for extruding and sacking the waste. Two circuits start from the steam chamber, one for water and the other for process steam, which means that there are two outlets, one towards the cartridge and the other towards the sterilization chamber.

The interior of the sterilization chamber which is reached through the intake for the cartridges, is occupied by a set of mechanisms which will be described later and which are responsible for treating the waste.

On situating the cartridge at the intake to the unit, the first operation which is carried out is that of opening the two automatic trapdoors or gates, with which the bag contained in the cartridge falls into the sterilization chamber.

Just below this intake is situated a grinder, which takes in the bag and grinds or triturates the waste into small grain size particles. While this operation is taking place, steam is injected in order to clean both the cartridge and the grinder itself, following which the trapdoors or gates are closed.

The material, reduced to a grain size of 1 mm., is made up of all kinds of solid and viscous liquid materials, which fall towards a metal separator, arranged vertically below the grinder.

The separated material is transferred to two selective mills for metals and fabrics respectively, which carry out a selective micronization of the material, which is then transferred to a lower position, where a sterotor, the sterilization chamber itself, which is the object of Patent Application ES.P.9401992, is situated.

This sterotor fulfils the strict cycle of the autoclave, describing the known diagram of peaks, throughs, and flat areas in times, pressures and temperatures of saturated steam, in accordance with the regulations of the World Health Organization (W.H.O.). This item is essentially horizontal in position.

Once that the material has been received in the autoclave and properly treated, the new cartridge housed at the intake to the unit is treated in the same way until it reaches the said autoclave, all of which happens without any kind of depressurization of the unit.

Adjacent to the sterotor is situated an extrusion chamber, responsible for collecting the pure solids that contain a high degree of humidity, which are extruded here into suitable bags, whereas the liquids, which have been sterilized and suitably filtered, are poured automatically into the waste water circuit.

A civil work collection box is situated close to the unit in order to collect the liquids, water and condensed items, while at the same time the said unit is provided with a water and heat recovery circuit in order to save electrical energy.

As has already been stated previously, the areas for the cartridges and for grinding are subject to draw sterilization in a closed circuit in order to guarantee that all particles have been detached at the end of the grinding.

The extruded material is stored in bags, which are sealed shut by welding (weldsealed) just at the outlet from the extrusion chamber, so that the solid material is received in containers, being dealt with as solid urban waste which can be taken advantage of later.

The whole unit undergoes washing with blasts of cleaning steam, then with jets of water at a high temperature and, finally, another wash with blasts of steam, after which the installation is ready to carry out a new treatment process.

All these and other aspects of the invention will be appreciated in greater detail by referring to the attached sheets of drawings, which are provided for guidance only, and in which the following are shown:

Figure 1, shows a general cross-section of the invention.

Figure 2, represents an elevation of a container cartridge.

Figure 3, is an external view of the unit.

Figure 4, allows the form of the connection between the cartridge and the inlet of the unit to be clearly seen.

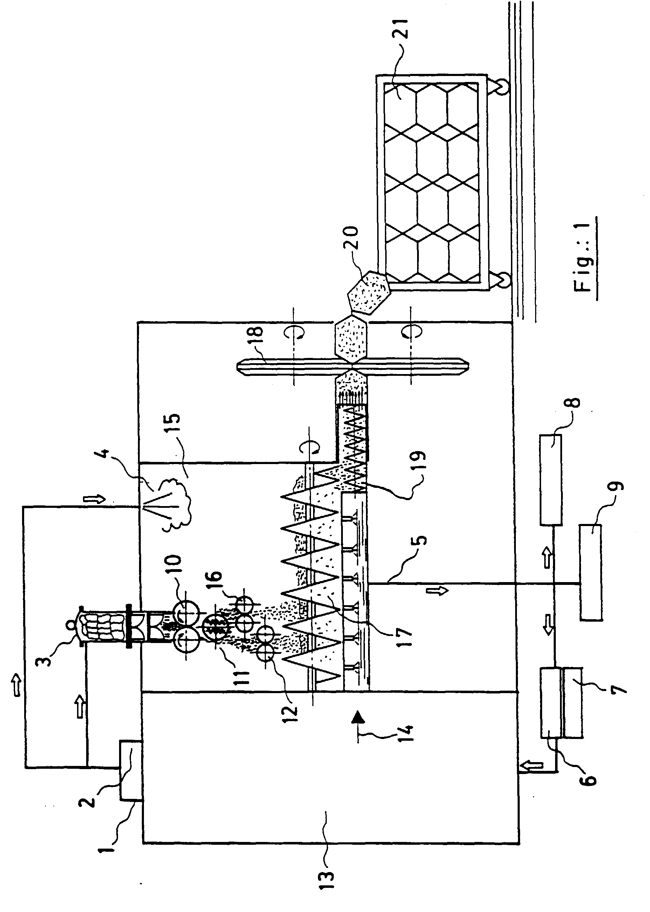

Looking now at Figure 1, the unit (26) can be appreciated, in which, starting from the steam boiler (13), the circuits for hot water for cleaning (1) and for steam (2) can be seen, being connected to the cartridge (3) and to the interior of the sterilization chamber (15) throug the intake (4). Below the intake into which the cartridge is inserted, the preliminary grinder (triturator) (10) can be seen, together with the metal separator (11) and the two selective mills for micronizing metals (16) and fabrics (12).

It can be observed that the micronized material falls towards the sterotor (14), which is arranged essentially in a horizontal position, below which the chamber for condensed items (14) is situated.

From this chamber (14), located outside the sterilization chamber (15), the pipe (3) evacuates all the liquids, sterilized and filtered, towards a drain (8) and towards a collection box, after passing through a station (9) for taking random samples for pathological analysis and connections for on-line measuring parameters and computerized recording.

Other points carry out a return of condensed items (6) and a return of washed items.

Inside the sterilization chamber (15) and to the right of the sterotor, we can see the extrusion chamber (19), at the outlet of which are situated tubular bags that are weldsealed at the ends into pre-set lengths at the welding machine (18), to form bags (20) which are deposited in containers.

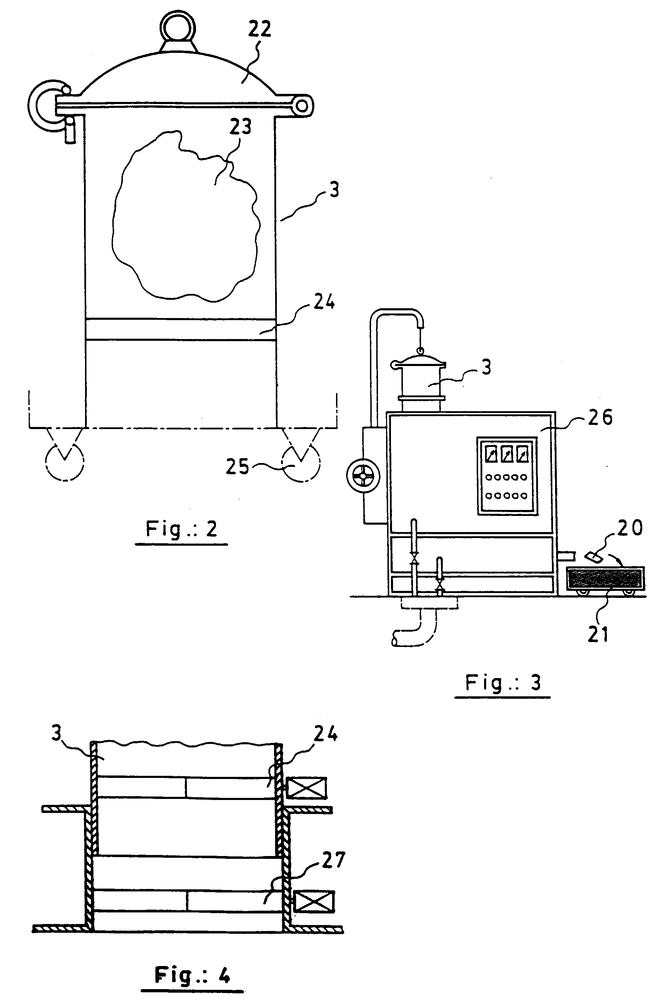

Each cartridge (3), Figure 2, is provided with a lid (22) with a hanging area, which is not numbered. The interior of the cartridges (3) is occupied, for instance, by red plastic bags or sacks weighing some 25 Kgs. which are used normally in hospital centres, in which unitary bags weighing some 2 Kgs. are housed. The lower connecting end shows its separated base (24), which is an automatic trapdoor or gate, for example a split butterfly gate, as well as a set of wheels for moving the cartridge.

By observing Figure 4, we can see the form of fast connection between the lower part of the cartridge and the intake into the unit, with the automatic trapdoor or gate (27) in the latter. Both trapdoors or gates are opened and/or closed in unison by means of any kind of conventional system.

It is important to point out, once having described the nature and advantages of this invention, its non-restrictive character, inashmuch as changes in the shape, materials or dimensions of its constituent parts will not in any way alter its essence, as long as they do not mean a substantial variation of the whole assembbly.

## Claims

1. System for treating hospital waste, starting from bags of the said waste, which is characterized by the following stages:
- housing the bags of hospital waste (23) in the interior of cylindrical cartridges (3) provided with wheels (25) at their lower end for movement, and being open at a certain height from their lower end and an automatic trapdoor or gate (24) being situated at this height,
- placing the cartridge into the upper cylindrical intake of the sterilizer unit (26), which is also provided with an automatic internal trapdoor or gate (27), with a hermetic fit and fast connection,
- opening in unison of the two automatic trapdoors or gates (24,27) in order to allow the bags to fall into the interior of the unit (26),
- grinding (trituration) of the bags and the waste they contain,
- providing steam from the interior of the unit (26) to the empty interior of the cartridge (3) in order to clean it, as well as to the grinder (triturator) (10),
- passing the ground waste through a metal separator (11),
- passing the metallic parts through a micronizer mill (16),
- passing the non-metallic parts through another micronizer mill (12),
- housing all the ground micronized waste in an autoclave in the interior of the unit (17), where it is kept slightly pressurized and desuperheated,
- repeating these operations until the autoclave contains a preset quantity of material,
- operating the autoclave or its specific function on the waste contained inside it,
- collecting, in a chamber situated below the autoclave, the liquids and condensed items for later treatment,
- transferring the product obtained from the autoclave to an extrusion chamber (19), in the interior of the sterilization chamber (15),
- housing or storing the product in bags, with the full bags then passing through a welding machine which seals the bags,
- evacuation of the said full bags towards the exterior of the unit.

2. System for treating hospital waste, starting from bags of the said waste, in accordance with claim 1, characterized in that the grinder (triturator) (10), the metal separator (11) and the micronization mills (12,16) are to be found situated vertically in relation to the intake for the cartridge and in the interior of a sterilization chamber housed inside the unit, in that the autoclave is also housed in this said sterilization chamber below the above named items and in an essentially horizontal position and direction towards the extrusion chamber.

3. System for treating hospital waste, starting from bags of the said waste, in accordance with claim 1, characterized in that a steam boiler (13) and the area for weld-sealing (18) the bags containing the inert waste are located in the unit outside the sterilization chamber (15).

4. System for treating hospital waste, starting from bags of the said waste, in accordance with claim 1, characterized in that the outlet (5) for the condensed items and liquids is derived towards a outlet of the unit in one portion for pouring towards a collection box (8) and in the other portion for returning (6,7) to the steam boiler (13).

5. System for treating hospital waste, starting from bags of the said waste, in accordance with claim 1, characterized in that both the automatic trapdoor or gate (24) in the cartridge and that (27) at the intake into the unit are of the split butterfly type, with these cartridges being provided with an upper lid (22) in order to allow the bags of waste to be inserted.
